# EUROPEAN PATENT APPLICATION

(11) **EP 4 819 151 A1**
(43) Date of publication of application: **07.10.2026**
(21) Application number: 25167741.5
(22) Date of filing: 01.04.2025
(51) Int. Cl.: G16H 20/70, G06F 21/31, G06F 21/32, G06F 40/20, G06N 3/006, H04L 9/32

(54) **AI-DRIVEN SYSTEM AND METHOD FOR POSTHUMOUS ADAPTIVE AI TWIN EVOLUTION, QUANTUM-SECURED LEGACY PRESERVATION, AND MULTI-GENERATIONAL THERAPEUTIC INTERACTION WITH REGRESSION THERAPY**

(71) Applicant: Wiem, Chiha, 9790 Petegem-Aan-De-Schelde (BE)
(72) Inventor: WIEM, Chiha, 9790 PETEGEM-AAN-DE-SCHELDE (BE); CHOKRI, Mehmud, 4021 Sousse (TN)

(57) **Abstract**

An Al-driven system and method for dynamic digital legacy preservation and multi-generational knowledge transfer, addressing the challenge of secure, emotionally intelligent legacy preservation across generations.

The system integrates Adaptive Al Twins, a Quantum-Secured Legacy Vault with lattice-based cryptography, and virtual reality (VR) environments to enable posthumous Al evolution, milestone-based memory unlocking, and therapeutic memory retrieval. Key features include an Al-Guided Emotional Memory Structuring System for subconscious memory retrieval using NLP-based regression therapy, an Al-Driven Multi-Generational Knowledge Transfer System for real-time holographic interactions, and accessibility interfaces (e.g., EEG neuro-interfaces) for inclusive use.

The system evolves through a continuous framework, from memory capture to a Mournalverse ecosystem, and supports lifelong Al Twin companionship for emotional coaching. It facilitates therapeutic memory retrieval, provides real-time mentorship, and ensures long-term legacy preservation, with scalability for future technologies like brain-computer interfaces.

## Description

### 2. Field of the Invention

The present invention pertains to the technical field of artificial intelligence (AI) systems for hybrid life journaling, dynamic digital legacy preservation, and multi-generational knowledge transfer and therapeutic interaction.

Specifically, it provides a system and method for generating and evolving an Adaptive AI Twin that facilitates posthumous evolution through real-time descendant interactions, integrating milestone-based data structuring, Al-driven contextual memory modeling with therapeutic regression therapy, quantum-secured storage using lattice-based cryptography, virtual reality (VR)-assisted therapeutic engagement, and holographic simulations for cross-generational mentorship (**Claim 1(a), Claim 1(b), Claim 1(c), Claim 1(d)**).

The invention employs AI-driven cognitive data processing, including natural language processing (NLP) and sentiment analysis, alongside biometric authentication (e.g., fingerprint, facial recognition) and AI-driven ethical governance to ensure privacy and cultural sensitivity, enabling secure, interactive, and dynamically evolving digital legacies that adapt across generations, overcoming the limitations of static digital afterlife archives (**Claim 1(e), Claim 2, Claim 8, Claim 11**).

By combining these technologies, the invention addresses the technical challenge of preserving and evolving family wisdom while fostering therapeutic, ethically governed interactions across time, advancing the field of AI-driven digital inheritance. An overview of the AI-driven ecosystem for these functionalities is provided in.

### 3. Background Art

Existing digital legacy preservation systems, including traditional journaling methods, pre-recorded messages, and social media archiving platforms, primarily rely on static data storage, which does not support adaptive evolution, interactive engagement, or recipient-specific personalization (**Claim 1(a)**, **Claim 10, Claim 15).** These conventional systems are designed for data preservation rather than ongoing relational and emotional continuity, limiting their ability to address the evolving needs of future generations.

Some digital afterlife solutions, such as EP3456789, employ AI-based simulation techniques using pre-existing user data. However, these approaches rely on fixed, non-evolving content that lacks real-time contextual adaptation, milestone-based retrieval, or AI-driven emotional calibration, as introduced in Claim 4 and Claim 11. While these models provide static reconstructions, they do not incorporate posthumous AI evolution that refines interactions based on descendant engagement over time.

Security remains a key challenge in digital legacy systems. Many existing platforms utilize standard cryptographic security, which may become vulnerable to quantum computing threats. The present invention addresses this limitation by incorporating quantum-resistant encryption (**Claim 1(b)**), biometric authentication (**Claim 3**), and tamper-proof access governance (**Claim 14**) to ensure long-term integrity and privacy protection.

Additionally, conventional systems often do not include ethical AI governance frameworks that regulate posthumous AI behavior, enforce user-configurable content access controls, or apply AI-driven cultural sensitivity screening to prevent unintended disclosures (**Claim 1(e), Claim 2, Claim 8**).

Existing VR memory reconstruction technologies, such as those described in EP2987654, facilitate immersive memory playback for therapeutic use. However, these approaches generally focus on predefined content delivery, lacking AI-driven emotional adaptability, subconscious memory retrieval, and multi-generational contextual evolution (**Claim 5, Claim 7, Claim 13**).

The present invention introduces an Adaptive AI Twin system that enables dynamic legacy preservation by continuously evolving posthumously, integrating AI-driven emotional intelligence, Quantum-Secured storage (Legacy Vault), and structured milestone-based content release (**Claim 1(a), Claim 1(b), Claim 1(c)**). By leveraging real-time sentiment analysis, predictive milestone-based AI learning, and immersive Virtual Reality(VR)-based therapeutic engagement, the system fosters an interactive, emotionally intelligent, and evolving legacy experience that preserves the user's authentic presence beyond conventional static archives.

The invention further enhances personalized memory structuring and emotional connection through an Al-assisted process that guides users through their past, present, and future, supporting self-reflection, intergenerational mentorship, and emotional healing (**Claim 5, Claim 9, Claim 11**).

This approach, using AI-driven voice, facial, and emotional synthesis pre-authorized by the user, enables a genuine and dynamic relationship between the user's Adaptive Al Twin and their descendants, ensuring that legacy preservation evolves as a meaningful, adaptive, and ethically governed process across generations (**Claim 1(a), Claim 2**).

Unlike prior art systems that apply these technologies in isolation (e.g., quantum security for data storage, Al for chatbot-based interactions, VR for entertainment), the present invention combines these elements into a unified ecosystem that facilitates emotionally intelligent legacy transfer, structured multi-generational mentorship, and secure, long-term data integrity (**Claim 8, Claim 15**).

This technological synergy enables a holistic approach to digital inheritance, memory preservation, and intergenerational emotional support, as further detailed in Sections 5 and 6 of the detailed embodiments.

Additionally, the invention is designed to scale across future advancements in quantum computing, VR, and AI-driven legacy interactions, ensuring long-term adaptability and seamless integration with emerging technologies.

### 4.1. Novelty & Differentiation

### 4.1.1 Prior Art & Technical Differentiation

The disclosed invention significantly advances digital legacy preservation by introducing a unified Al-driven ecosystem designed for dynamic, multi-generational legacy evolution and interactive posthumous engagement (**Claims 1-15**). Unlike existing digital afterlife solutions that rely on static data archival, non-adaptive AI interactions, or isolated technologies, the present invention employs an emotionally intelligent Adaptive AI Twin, structured milestone-based content delivery, and immersive, therapeutic memory reconstruction, resulting in a secure, continuously evolving, and contextually responsive digital legacy experience.

The invention substantially differentiates from known prior art, which applies these technologies in fragmented, non-synergistic ways:

### Existing AI Chatbot-Based Memorial Models

- social media posts, transcripts) generate legacy personas, but lack adaptive multi-generational learning, real-time emotional calibration, or structured milestone-triggered content delivery.
- Mournal's Novelty: Implements an Adaptive AI Twin that evolves posthumously using real-time sentiment analysis, predictive emotional modeling, and subconscious regression therapy, expected to achieve a 30% increase in response emotional accuracy and contextual depth across successive generations based on the performance of sentiment analysis and regression therapy (pending further validation) (**Claim 1(a), Claim 3, Claim 8**).

Unlike conventional chatbots, Mournal's Adaptive AI Twin actively learns from descendant interactions using machine learning, ensuring a dynamic, evolving presence.

The workflows for the Adaptive AI Twin's posthumous evolution are further detailed in Section 5.4.

### Existing Pre-Recorded Audio & Video Legacy Models

- Existing Approach: Pre-recorded messages and video archives are delivered at predefined life events (e.g., marriage, graduation) without emotional adaptability or recipient-specific psychological readiness detection, which can result in emotional dissonance where messages feel premature, untimely, or overwhelming for the recipient.
- Mournal's Novelty: Implements AI-driven emotional readiness calibration, ensuring messages are ethically delivered when the recipient is emotionally prepared. Eva's Sentiment-Based AI Learning analyzes the recipient's real-time emotional state (e.g., facial cues, vocal tone, text sentiment analysis) using NLP and sentiment analysis before triggering content release, preventing emotional distress by adjusting message tone, timing, and co ntextual framing.

The Milestone + Readiness Dual-Layer Protection ensures legacy messages are released gradually when truly needed, making Mournal the first adaptive Al-driven legacy system that personalizes content at both a milestone and psychological level (**Claim 1(b), Claim 1(e), Claim 4, Claim 11, Section 5.7**).

### Existing AI-Driven Virtual Companions

- Existing Approach: AI-driven virtual companions focus on simulated companionship for living users, but lack structured legacy transmission, generational knowledge evolution, or secured multi-generational interactions.
- Mournal's Novelty: Establishes a multi-generational AI knowledge network, where AI Twins integrate regression therapy-derived emotional context and continuously evolve across generations using NLP and machine learning, providing personalized, posthumous mentorship authenticated via biometric heir verification (**Claim 1(a), Claim 4, Claim 5, Section 5.6**).

### Existing Digital Immortality Platforms

- Existing Approach: Passive recording of social interactions creates static digital replicas, but these models lack structured milestone-based delivery, adaptive AI evolution, and quantum-secured storage, making them susceptible to long-term integrity risks.
- Mournal's Novelty: Combines milestone-triggered message release, quantum-secured legacy vaults, and neural-network-driven emotional modeling to deliver an adaptive, privacy-compliant, and context-aware posthumous AI presence, expected to achieve 99.9% tamper-proof data integrity via lattice-based cryptography and quantum key distribution (QKD) (pending further validation) (**Claim 1(b), Claim 3, Claim 14, Section 5.7**).

### Existing VR & Interactive Legacy Playback Systems

- Existing Approach: VR-based memorials offer passive playback of recorded memories, lacking Al-driven adaptation, emotional intelligence processing, or descendant-driven dynamic interactions.
- Mournal's Novelty: Leverages VR-based cognitive regression therapy, AI-powered holographic storytelling, and real-time emotional adaptation using sentiment analysis to enable interactive reliving of legacy moments, integrating AI-driven memory structuring and emotional adaptability **(Claim 1(c), Claim 1(d), Claim 9, Claim 15, Section 5.9).**

### 4.1.2 Summary of Key Novel Distinctions of Mournal

Adaptive Emotional Intelligence and Generational Evolution: Utilizes sentiment analysis and regression therapy to create emotionally intelligent, generationally adaptive AI Twins that evolve through family input, providing enhanced emotional depth and adaptability compared to existing solutions (**Claim 1(a), Claim 3, Claim 5, Claim 15**).
- **Structured Milestone-Based Delivery:** Automated, recipient-specific unlocking of legacy content triggered by milestone life events, secured by biometric and quantum-secured cryptographic measures (**Claim 1(b), Claim 4, Claim 14**).
- **Immersive Interactive Holographic Engagement:** Real-time adaptive holographic avatar interactions using machine-learning techniques, enhancing cross-generational legacy engagements (**Claim 1(d), Claim 9**).
- **Advanced Accessibility and Inclusivity:** Comprehensive integration of biometric authentication, eye-tracking, voice-command, and EEG neuro-interface technologies, ensuring broad usability for individuals with impairments (**Claim 7, Claim 13**).
- **Configurable Privacy for Locked Content:** Allows users to designate private content (e.g., self-reflection journals, healing videos) that remains inaccessible to descendants but informs adaptive AI Twin's development, enhancing the AI Twin's emotional authenticity by incorporating private emotional data while protecting privacy (**Claim 1(a), Claim 1(e), Claim 12**).
- **Culturally Sensitive, Ethically Governed AI:** Integrates AI-driven cultural sensitivity screening and ethical governance that adapts to evolving societal norms, ensuring legacy content remains respectful across generations (**Claim 1(e), Claim 12, Claim 15**).
- **Lifelong AI Twin Companion for Child Development:** Extends the adaptive AI Twin's role to grow with a child from birth, providing Al-driven mentorship and emotional support to foster resilience and personal growth (**Claim 1(a), Claim 10, Claim 15**).

The combination of predictive AI-driven milestone delivery (**Claim 3**) with real-time biometric emotional analysis (**Claim 8**) is not an expected evolution of existing legacy vaults or AI chatbots, as standard AI memorials remain static and milestone-based vaults lack emotional intelligence. This synergy enables a novel technical effect: adaptive, emotionally intelligent content delivery that adjusts to the recipient's emotional state, which static systems cannot achieve.

Thus, the invention provides a novel, integrated solution for legacy preservation, advancing the field through secure, adaptive, therapeutic, and lifelong supportive interactions across generations.

### 5. Detailed Description of the Invention

### 5.1 Overview

The invention provides a system for preserving and evolving emotional legacies across generations, integrating AI-driven emotional intelligence, quantum-secured storage, VR-based therapeutic engagement, cross-family knowledge sharing, and accessibility features to ensure inclusive, secure, and emotionally resonant interactions using sentiment analysis and emotional adaptability (**Claim 1, Claim 4**).

Existing AI chatbots, which simulate deceased individuals using pre-recorded data, generally lack the ability to evolve posthumously through multi-generational engagement; for example, a known system (EP3456789) relies on static data, limiting its emotional authenticity and long-term relevance for descendants.

Similarly, VR memory reconstruction systems, such as those enabling memory reliving (e.g., EP2987654), do not integrate AI-driven emotional processing for therapeutic outcomes, restricting their utility in emotional healing. Quantum encryption techniques, widely documented in prior art publications, secure data effectively but fail to address the challenge of emotionally appropriate content delivery tailored to recipients' readiness.

Mournal solves these problems by combining an Adaptive AI Twin with posthumous evolution, quantum-secured milestone access, and VR-based therapeutic engagement, enabling a dynamic, secure, and emotionally intelligent legacy preservation system across generations.

The accessibility features (**Claim 7**) ensure that all users, regardless of physical or cognitive abilities, can interact with the AI Twin, thereby enabling inclusive multi-generational engagement; a critical aspect of preserving legacies across diverse family members.

Similarly, the cross-family knowledge sharing system (**Claim 6**) allows the AI Twin to refine familial wisdom by integrating insights from separate lineages using NLP and machine learning, enhancing the system's ability to provide contextually adaptive, multi-generational guidance (**Claim 5**).

### 5.1.1 Limitations of Existing Systems & Technical Problem

Existing Digital Legacy Solutions: Static, Fragmented, and Non-Evolving: Current digital legacy and memorialization systems exhibit significant limitations in their ability to preserve, evolve, and securely transfer emotional and cognitive legacies across multiple generations. These existing solutions can be classified into the following categories:

### AI Chatbot-Based Memorials (Static AI Clones)

- Operate on passively scraped social media content or pre-recorded messages, offering non-adaptive and contextually rigid interactions.
- Lack posthumous adaptability, resulting in AI responses that remain unchanged over time, failing to reflect the evolving emotional and cognitive needs of descendants.

### Pre-Recorded Audio & Video Legacy Models

- Deliver predefined, static messages at fixed life milestones (e.g., marriage, graduation) without emotional adaptability or recipient-specific psychological readiness detection.
- Lack AI moderation, which may lead to emotionally misaligned or distressing experiences for recipients.

### Traditional VR Memory Reconstruction Systems

Provides passive playback of pre-recorded content, rather than dynamically reconstructing and adapting emotional states based on descendant interactions, wherein they:
Do not incorporate Al-driven emotional recalibration, preventing real-time adjustment of experiences based on user sentiment analysis.

### Standard Digital Archives & Blockchain-Based Legacy Vaults

Stores legacy content but lacks structured, milestone-based content release mechanisms that ensure access is aligned with the recipient's emotional state.

Existing security models, such as RSA-4096 and AES-256 encryption, are vulnerable to future quantum computing advancements, exposing legacy data to long-term security risks.

These limitations result in detached, non-adaptive, and insecure digital legacies that fail to preserve emotional authenticity, ensure secure generational knowledge transfer, or dynamically engage with descendants in a meaningful way.

### 5.1.2 Journal's Technical Innovations: A Paradigm Shift in Generational Legacy Preservation

Mournal addresses these deficiencies through an integrated AI-driven framework that ensures posthumous AI evolution, quantum-secured storage, immersive VR therapy, and ethical AI governance to create an adaptive, emotionally intelligent, and ethically safeguarded legacy system.

### 5.1.2.1 Posthumous AI Evolution: Adaptive AI Twin Learning Over Generations

Mournal's Adaptive AI Twin continuously refines itself posthumously, integrating real-time descendant interactions, sentiment analysis, and generational context updates to ensure ongoing legacy evolution (**Claim 1(a), Claim 10, Claim 13**).
→ a Quantum Adaptive Learning Engine, a machine-learning framework that uses quantum-secured storage to securely store, refine, and adjust AI Twin responses across generational shifts, ensuring data integrity during learning processes.
→ *Advancement over Prior Art:* Unlike conventional AI memorials, which operate on static, pre-recorded inputs, Mournal enables a continuously learning and evolving AI presence, maintaining contextual relevance across multiple generations.

### 5.1.2.2 Quantum-Secured Legacy Vault: Future-Proof, Tamper-Resistant Archival

Utilizes lattice-based cryptography and the BB84 protocol, which uses quantum key distribution to detect eavesdropping via quantum states, to prevent quantum decryption threats, ensuring legacy data integrity for extended periods (**Claim 1(b), Claim 3, Claim 14**).
→ Biometric-controlled access (fingerprint, facial recognition, blockchain key verification) ensures that only authorized heirs can retrieve and interact with stored content.
→ Advancement over Prior Art: Existing legacy vaults rely on classical encryption, which will become obsolete in the post-quantum era. Mournal ensures long-term data security with quantum-resistant methodologies.

### 5.1.2.3 AI-Driven VR Memory Reconstruction: Therapeutic Multi Generational Engagement

Integrates VR-based subconscious memory recall using 3D rendering, spatial audio, and AI-driven regression therapy, allowing descendants to relive past experiences through AI-driven memory synthesis (**Claim 1(c), Claim 1(d), Claim 9, Claim 15**).
→ AI-guided emotional calibration, using sentiment analysis and regression therapy, dynamically adjusts tone, visual elements, and environmental factors to optimize therapeutic impact, facilitating emotional healing and intergenerational bonding.
→ *Advancement over Prior Art:* Existing VR-based memory systems provide passive playback and lack adaptive emotional processing. Mournal's AI-powered real-time sentiment analysis ensures tailored legacy experiences that support emotional healing and intergenerational bonding.

### 5.1.2.4 Biometrically-Governed AI Ethical Content Release

Mournal's system ensures biometrically authenticated access, verifying heirs before unlocking content, which is structured by context-sensitive, ethically governed release protocols (**Claim 1(b), Claim 1(e), Claim 12**).
→ Milestone-based release (e.g., wedding, first child, career transition) ensures that content is unlocked only when the recipient is emotionally prepared to receive it, as determined by sentiment analysis and emotional readiness calibration.
→ *Advancement over Prior Art:* Competing digital archives lack emotional verification and release content arbitrarily, which may result in unintended psychological distress. Mournal introduces AI-calibrated, milestone-sensitive content governance, ensuring ethically responsible posthumous knowledge transfer.

### 5.1.2.5 AI-Driven Predictive Generational Mentorship

Provides personalized guidance based on descendant life-stage adaptation and real-time sentiment analysis, ensuring AI Twins offer context-aware, emotionally relevant mentorship over time (**Claim 10, Claim 11, Claim 15**).
→ Implements an AI-powered societal adaptation engine, which uses NLP and machine learning to analyze societal trends and adjust the AI Twin's guidance according to evolving cultural and generational norms.
→ *Advancement over Prior Art:* Existing AI-driven digital afterlife solutions provide static responses that become outdated. Mournal ensures ongoing wisdom refinement through a dynamic Al Twin evolution process.

### 5.1.2.6 Multi-User AI Collaboration & Living Ancestry Tree

Mournal facilitates multi-user AI Twin collaboration, allowing multiple family members to jointly contribute, refine, and expand generational wisdom within an evolving, AI-driven digital repository (**Claim 1(d), Claim 15**).

Additionally, the system incorporates a cross-family knowledge exchange framework, enabling heirs to ethically integrate and refine ancestral insights across separate lineages. Differential privacy protocols are utilized to safeguard sensitive family data and ensure ethical data integration.
→ *Advancement over Prior Art:* Traditional genealogy databases provide static records without interactive or adaptive generational engagement. Mournal advances beyond conventional platforms by offering a collaborative, ethically governed AI-driven legacy system that evolves dynamically based on ongoing family input and interaction.

### 5.1.3 Conclusion

Unlike passive digital afterlife solutions, which rely on pre-recorded messages, fixed chatbot models, or legacy vaults vulnerable to quantum threats, Mournal introduces a living, evolving, ethically governed AI Twin secured through quantum encryption, AI-calibrated emotional intelligence, and immersive VR therapy. This system represents a significant technological advancement by integrating:
- Posthumous AI evolution, ensuring continuous legacy adaptation over time.
- Quantum-secured storage, providing long-term data protection beyond conventional encryption.
- VR-based memory reconstruction, delivering emotionally calibrated, AI-driven immersive legacy experiences.
- Ethical AI governance, ensuring that posthumous content release is secure, responsible, and emotionally appropriate through sentiment analysis and emotional readiness calibration.

By combining adaptive AI learning, milestone-based legacy unlocking, predictive mentorship, and quantum-secured privacy, Mournal establishes the first-ever AI-driven, emotionally intelligent, and ethically safeguarded digital legacy system. This technological framework fundamentally redefines the concept of legacy preservation, ensuring that emotional wisdom, ethical values, and multi-generational knowledge remain dynamic, contextually relevant, and securely transmitted across time.

### 5.2 Core Components of the Mournal System

Mournal's system comprises several core components that work synergistically to achieve dynamic, multi-generational legacy preservation, as described in Claims 1-15.

### 5.2.1 Hybrid Journaling System

Captures family knowledge data through physical (OCR-digitized), digital (text, audio, video), and VR inputs, processed by Eva's six-engine AI framework;Conversational NLP (e.g., BERT/GPT), Emotional Intelligence, Regression Analysis, Memory Structuring, Ethical Governance, and Predictive Milestone;to ensure dynamic memory structuring, unlike static storage systems that lack emotional depth (**Claim 1(a)**).

### 5.2.1.1 Three Main Jobs of the Hybrid Journaling System

The Hybrid Journaling System serves three primary functions that fulfill Mournal's mission: facilitating emotional healing through AI-guided therapeutic processing of psychologically distressing or unresolved past experiences; preserving multi-generational wisdom through secure, milestone-based legacy content capture; and continuously refining the AI Twin's emotional authenticity through adaptive learning algorithms and sentiment-driven calibration.
- **Therapeutic Emotional Processing:** Utilizes AI-guided prompts and regression therapy methodologies to facilitate emotional healing by guiding users through the structured reflection of past experiences, contextual understanding of present identity, and envisioning future goals. For example, a user might journal about a childhood memory of loss, with Eva providing targeted prompts to process unresolved grief and enhance emotional closure **(Claim 5, Claim 11).**
- **Multi-Modal Legacy Preservation:** Captures authentic user presence through multi-modal inputs (text, audio, video, VR), ensuring emotionally adaptive content structuring, experiential authenticity, and milestone-specific secure archival for subsequent generations. For instance, a user might record a milestone-specific video message for their grandchild's graduation, securely stored for emotionally calibrated future release (**Claim 1(a), Claim 4**).
- **Adaptive AI Twin Emotional Calibration:** Processes journaled content to dynamically train and calibrate the Adaptive AI Twin, employing natural language processing, sentiment analysis, and emotional intelligence algorithms. Locked private content further enhances the AI Twin's emotional authenticity without disclosure to heirs, thereby ensuring adaptive emotional fidelity across generations (**Claim 1(a), Claim 1(e)**).

### 5.2.2 Adaptive AI Twin

A digital representation mirroring user personality, evolves through interconnected AI modules (e.g., reinforcement learning for adaptation, sentiment analysis for emotional calibration), expected to achieve a 30% improvement in emotional response accuracy based on sentiment analysis and regression therapy, pending further validation, addressing the lack of posthumous evolution in existing AI systems (**Claim 1(a), Claim 3, Claim 8**).

### 5.2.3 Quantum-Secured Legacy Vault

Utilizes quantum-resistant encryption (e.g., lattice-based cryptography, BB84 protocol) to secure data against quantum computing threats, expected to achieve 99.9% tamper-proof integrity based on these technologies, pending further validation-a novel application for legacy preservation surpassing traditional encryption methods (**Claim 1(b), Claim 12**).

The vault integrates biometric access control (fingerprint, facial, voice authentication) and privacy controls (opt-in/opt-out permissions, heir-specific visibility) to prevent unauthorized access, addressing privacy gaps in existing systems (**Claim 1(b), Claim 1(e)**).

### 5.2.4 VR-Based Memory Reconstruction Module

Provides immersive VR environments (e.g., Momverse) for therapeutic memory exploration, integrating AI-driven regression therapy with 3D rendering and spatial audio to facilitate emotional healing, a feature absent in conventional VR systems (**Claim 1(c), Claim 5**). The VR environments for memory reconstruction, such as Momverse.

### 5.2.5 Ethical AI Governance Engine (Eva)

Conducts automated audits using differential privacy and AI-driven ethical monitoring, ensuring compliance with user-defined policies, cultural sensitivity, and regulatory standards (Claim 1(e)). Eva's six-engine architecture, including Conversational NLP, Emotional Intelligence, Memory Structuring, Regression Learning, Predictive Calibration, and Quantum Evolution.

For example, a user's journal entry is processed through Eva's Conversational NLP to extract emotional context, encrypted and stored in the Quantum-Secured Legacy Vault, and later reconstructed in VR for a descendant.

Eva dynamically adjusts tone and delivery based on the descendant's emotional state , showcasing the non-obvious integration of AI-driven memory structuring, secure storage, and therapeutic VR presentation.

This synergistic framework ensures Mournal not only preserves multi-generational wisdom, but also enables emotionally intelligent, secure, and ethically governed interactions across time.

### 5.3 Functional Objectives of the Mournal System

Cross-generational knowledge sharing is enabled through AI Twins that provide real-time mentorship using transformer-based NLP (e.g., BERT/GPT), ensuring context-aware guidance for descendants, unlike static legacy tools (**Claim 15)**.

Accessibility and inclusivity are ensured through multi-modal inputs (e.g., eye-tracking, EEG), supporting users with impairments, while therapeutic audio modules (e.g., 432 Hz binaural beats) enhance emotional well-being, addressing gaps in conventional systems (**Claim 7, Claim 13**). Secure and ethical preservation is achieved through quantum-resistant encryption (lattice-based cryptography, BB84 protocol) and AI-governed data access, ensuring long-term integrity and tamper detection (**Claim 1**(**e**), **Claim 12**).

For example, a message recorded by a parent is ethically delivered to their child on a milestone event (e.g., wedding), with Eva adjusting tone and delivery timing based on the child's real-time emotional readiness (**Claim 1(e)**).

This integration of emotional intelligence, secure vaults, milestone delivery, and accessibility tools exemplifies Mournal's architecture for ethically calibrated, cross-generational interaction.

### 5.4 AI Twin Development and Evolution

Mournal's Adaptive AI Twins create an emotionally intelligent, perpetually evolving familial presence using sentiment analysis and reinforcement learning, as described in **Claim 1(a), Claim 1(d), Claim 9,** and **Claim 11.** Unlike existing AI systems that remain static after deployment, Mournal's AI Twins actively learn and refine their responses through a multi-stage process. Initial modeling captures family knowledge data (text, voice, video, subconscious memories via NLP-based regression therapy) using Eva's Conversational NLP (e.g., BERT/GPT) and Emotional Intelligence Engine to replicate user-specific patterns, such as a mother's nurturing tone (**Claim 1(a)**).

Emotionally responsive calibration adjusts voice tone, speech cadence, and emotional expressiveness using real-time sentiment analysis and behavioral modeling, analyzing inputs like vocal tone, text, and facial expressions, to enhance emotional response accuracy through sentiment analysis and regression therapy, ensuring empathetic interactions (**Claim 3, Claim 8, Claim 11**) Studies on sentiment analysis in NLP applications have reported accuracy improvements of 25-38% in emotional recognition [1], supporting Mournal's approach.

Generational evolution integrates descendant interactions into a graph database (e.g., Neo4j), using reinforcement learning to refine emotional depth, enabling the AI Twin to provide context-sensitive guidance, such as resilience advice to a descendant facing a challenge, a feature absent in conventional systems (**Claim 1(d), Claim 9**).

Ethical compliance is ensured through biometric authentication and quantum-resistant encryption, with Eva's Ethical AI Governance Engine using differential privacy to safeguard privacy and cultural sensitivity, addressing gaps in existing systems (**Claim 1(e), Claim 2**).

### 5.4.1 AI Twin Evolution

The Adaptive AI Twin distinguishes itself from static AI memorial models by employing reinforcement learning, sentiment analysis, and milestone-based contextual adaptation to evolve posthumously through descendant interactions. Unlike existing digital legacy systems, which rely on pre-recorded messages or static personality clones, the AI Twin dynamically refines its responses based on generational engagement (**Claim 1(a)**).

The integration of posthumous AI evolution (**Claim 1(a)**), quantum-secured milestone access (**Claim 3**), and VR-based therapeutic engagement (**Claim 4**) addresses a key limitation in prior art: the inability of legacy AI models to maintain emotional depth and contextual relevance across generations.

By leveraging predictive emotional AI learning, the system detects user sentiment and milestone triggers (e.g., significant life events such as a descendant's coming of age) to adapt legacy content delivery accordingly.

The AI Twin's capability to enhance response accuracy and emotional appropriateness is supported by existing research in adaptive AI dialogue modeling and reinforcement learning-based personality evolution.

By refining response calibration through user feedback loops, the AI Twin achieves a higher level of contextual adaptation and sentiment-aware accuracy than conventional models, ensuring an emotionally intelligent and dynamically evolving posthumous presence.

### 5.4.2 Technical Example of Posthumous Evolution

A user records a message about overcoming adversity, which the AI Twin initially uses to provide general encouragement. Over time, as descendants interact with the AI Twin, it learns from their emotional responses (e.g., a descendant's anxiety about a career change) and refines its advice, incorporating new family insights (e.g., a sibling's story of career success) to offer tailored guidance, such as, "Your uncle faced a similar challenge and succeeded by networking; here's how you can do the same."

This continuous learning, driven by reinforcement learning and sentiment analysis, ensures the AI Twin evolves posthumously, distinguishing it from static chatbots (**Claim 1(a), Claim 10, Claim 13**,).

For example, an AI Twin evolves to offer parenting advice to a descendant, drawing on new family interactions stored securely in the Quantum-Secured Vault, enhancing the AI Twin's emotional authenticity, showcasing the non-obvious synergy of posthumous AI evolution, emotional intelligence, and ethical governance that makes Mournal a pioneering system for facilitating therapeutic cross-generational interactions.

### 5.5 Secure Legacy Vault & Ethical AI Management

Mournal's Secure Legacy Vault and Ethical AI Management system ensures secure, ethical, and privacy-compliant intergenerational knowledge sharing, as described in Claim 1(b), Claim 1(d), Claim 1(e), and Claim 12.

The Quantum-Secured Legacy Vault employs lattice-based cryptography and quantum key distribution (QKD) via the BB84 protocol to achieve a preliminary estimated 99.9% tamper-proof data integrity, based on the performance of these technologies in securing data against quantum attacks, pending further validation (**Claim 1(b), Claim 3**).
- Lattice-based cryptography provides quantum-resistant encryption by using mathematical structures that are computationally hard to break, even with quantum computers, while QKD ensures secure key exchange by detecting eavesdropping attempts through quantum principles.
- Biometric access control uses fingerprint, facial, and voice authentication to prevent unauthorized access, such as an heir verifying identity to access a video message (**Claim 1(e), Claim 12**).
- A hybrid AI-driven death verification system confirms the user's passing by analyzing official records and executor approvals, triggering posthumous legacy management.
- Privacy controls provide opt-in/opt-out permissions, heir-specific visibility, and restricted memory access (e.g., private for immediate heirs, semi-public for extended family), enforced via blockchain, addressing privacy gaps in existing systems (**Claim 1(d)**).
- Eva's Ethical AI Governance Engine conducts automated audits using differential privacy, ensuring compliance with user-defined constraints, such as anonymizing sensitive data during cross-lineage exchanges, a feature absent in conventional systems (**Claim 1(e)**).

### 5.5.1 Inventive Step of Milestone-Based Unlocking

The milestone-based content delivery releases content at verified life events (e.g., a wedding message), adjusted via Eva's Sentiment Analysis for emotional readiness, unlike static delivery in existing systems.

The combination of quantum security (lattice-based cryptography ensuring 99.9% tamper-proof integrity) and AI-driven emotional readiness calibration (assessing facial cues, vocal tone, and sentiment) creates a non-obvious synergy, as it ensures both security and emotional appropriateness, a technical advancement over pre-set message release systems (**Claims 4, Claim 12**).

For example, a user restricts a personal letter to their child while sharing a recipe cousins, with Eva ensuring ethical compliance and the Quantum-Secured Vault protecting the data, demonstrating the non-obvious synergy of quantum security, ethical AI, and adaptive delivery that makes Mournal a groundbreaking movement for preserving family wisdom with integrity and love.

### 5.6 Mournalverse & Multi-Generational AI Evolution

The Mournalverse is a multi-generational AI legacy ecosystem integrating all environments (Momverse, Dadverse) to facilitate cross-generational interaction and wisdom evolution, as described in Claim 1(d), Claim 1(e), Claim 9, Claim 12, and Claim 15.

Unlike existing avatar systems that offer static interactions, the Mournalverse provides a dynamic network through the Living Ancestry Tree, a graph database (e.g., Neo4j) populated by interconnected AI Twins, enabling descendants to engage with ancestors, such as a great-grandparent sharing family values, with responses tailored via Eva's Conversational NLP (e.g., BERT/GPT) and reinforcement learning, a feature not disclosed in conventional systems (**Claim 1(d), Claim 15**).

Real-time holographic interactions, using transformer-based models and quantum-secured storage, allow family members to interact with holographic AI Twins, adjusted for emotional states via Sentiment Analysis, expected to achieve a 30% improvement in emotional response accuracy based on sentiment analysis and regression therapy, pending further validation, surpassing static avatar interactions (**Claim 1(d), Claim 8, Claim 9**).

Cross-family knowledge expansion enables AI Twins to share wisdom across lineages using differential privacy, such as sharing a family tradition while protecting sensitive data, a novel application not found in existing systems (**Claim 1(d)**).

Future scalability includes quantum-enhanced holography and neuro-interface integration (e.g., Emotiv EPOC) for thought-driven interactions, enhancing accessibility (**Claim 1(d), Claim 7**).

For example, a family reunion in the Mournalverse features a holographic AI Twin sharing a story, with responses adapted to participants' emotions and data secured in the Quantum-Secured Vault, showcasing the non-obvious synergy of immersive engagement, emotional intelligence, and security that makes Mournal a system for facilitating therapeutic cross-generational interactions through shared wisdom.

### 5.6.1 Cross-Family System

The Cross-Family Al Twin Knowledge Expansion System enables collaborative refinement of familial wisdom across separate lineages (**Claim 6**). It directly supports the invention's goal of multi-generational legacy preservation by enabling the Al Twin to integrate diverse familial insights, ensuring the wisdom it provides remains relevant and enriched across generations.

### 5.7 VR-Based Immersive Legacy Exploration Environments

Mournal's VR-Based Immersive Legacy Exploration Environments (Momverse, Dadverse, Mournalverse) introduce an AI-integrated framework for cross-generational connection and therapeutic memory reliving, as described in Claim 1(c), Claim 1(d), Claim 5, and Claim 9. Unlike existing VR memorial systems that rely on passive playback of recorded memories, Mournal's environments dynamically adjust memory experiences based on user sentiment, cognitive state, and milestone-based interactions through an AI-driven Legacy Tree (graph database) and NLP-powered regression therapy.

The Momverse, designed for maternal legacy preservation, facilitates AI-guided therapeutic memory reliving using natural language processing (NLP) regression models (e.g., transformer-based BERT/GPT) to reconstruct past experiences while integrating emotional adaptation techniques. For instance, a user engaging with a memory of baking with their mother experiences context-sensitive AI moderation, where Eva adjusts sensory intensity and emotional framing in response to the user's real-time sentiment and physiological indicators, ensuring a controlled and emotionally supportive environment (**Claim 1(c), Claim 5**).

The Dadverse, a paternal-focused VR environment, enhances AI-driven milestone mentorship through holographic engagement. Within the Legacy Firepit, users receive adaptive mentorship messages from their AI Twin or ancestral holographic figures, leveraging long short-term memory (LSTM)-based NLP models to refine content based on emotional state and personal milestones. This enables dynamic career mentorship, fatherhood guidance, and wisdom sharing, evolving in relevance with each successive generation (**Claim 1(c), Claim 9**).

The Mournalverse expands upon these environments by enabling multi-generational VR engagement, where families can participate in real-time, AI-moderated interactions using holographic projections (e.g., light-field displays). Unlike static VR systems that do not evolve posthumously, the Mournalverse dynamically refines conversational interactions across generations through Eva's Sentiment Analysis and Adaptive AI Twin models, ensuring a progressively enriched familial knowledge base (**Claim 1(d), Claim 8**).

Future enhancements include neuro-interface integration (e.g., EEG-based Emotiv EPOC systems) to enable thought-driven interactions, enhancing accessibility for users with cognitive and physical impairments (**Claim 7**). This ensures inclusivity within the VR healing environments, reinforcing Mournal's role as a system for emotional healing, knowledge transfer, and multi-generational connection through Al-enhanced immersive engagement.

### 5.8 Advanced Inclusive Engagement Interfaces

Mournal's Advanced Inclusive Engagement Interfaces (Mournal Express) ensure accessibility for diverse users, as described in Claim 7 and Claim 13. Unlike conventional systems that lack accessibility features, Mournal's Multi-Modal Input System supports voice activation (e.g., Google Speech-to-Text API), eye-tracking (e.g., Tobii Eye Tracker), and EEG-based neuro-control (e.g., Emotiv EPOC), enabling users with impairments to journal, such as a hospice patient recording a message via voice activation, a novel application in legacy preservation (**Claim 7, Claim 13**).

The Healing Frequencies Module integrates therapeutic sound frequencies (e.g., 528Hz binaural beats) during regression therapy sessions, delivered via audio output synchronized with VR or journaling interfaces, to support emotional well-being, such as calming a terminally ill patient, a feature not disclosed in existing systems (**Claim 5**).

Mournal Express accelerates AI Twin creation within 24 hours using voice inputs and regression therapy, expected to achieve up to 50% faster deployment compared to conventional systems, pending further validation, addressing delays in legacy preservation (**Claim 1(a), Claim 13**).

Future enhancements include EEG-based controls (e.g., Emotiv EPOC) for thought-driven interactions, such as navigating VR environments or selecting journal prompts, further enhancing accessibility for users with severe impairments (**Claim 7**).

### 5.8.1 Accessibility Features

The accessibility features are a necessary technical contribution, as they enable generational AI interaction for users with impairments, ensuring equitable access to Mournal's therapeutic and legacy preservation capabilities. For example, a user with motor impairments can journal via eye-tracking, ensuring their legacy is preserved, which directly supports the invention's goal of inclusive multi-generational engagement (**Claim 7, Claim 13**).

The AI-powered accessibility module ensures inclusive user interaction with the AI Twin (**Claim 7**). It ensures that the AI-driven generational legacy preservation system is inclusive, allowing all family members to engage with the AI Twin and contribute to the multi-generational legacy, regardless of physical or cognitive limitations.

For example, a terminally ill user records a message via voice activation, with Eva guiding the process and integrating healing frequencies during regression therapy, demonstrating the non-obvious synergy of accessibility, therapeutic audio, and expedited AI Twin creation that makes Mournal a system for facilitating inclusive and therapeutic legacy preservation.

This inclusive design not only addresses accessibility gaps in prior art but introduces a non-obvious technical synergy: the combination of AI-guided journaling, therapeutic frequency modules, and voice/eye/neural input ensures that users with diverse cognitive and physical abilities can fully participate in the legacy-building process.

This integration elevates Mournal from an archival system to an ethically grounded, therapeutically inclusive legacy platform, where no story is left untold, and no voice is left unheard.

### 5.9 Continuous Evolution of Mournal and AI Twins

Mournal and its AI Twins evolve through a unified, continuously evolving AI-driven framework that progresses through an interconnected continuum of development, as described in Claims 1-15, surpassing static systems. This evolutionary continuum is not composed of distinct or separable stages but represents a progressive integration of capabilities, where each stage builds upon the previous one to culminate in the unified Mournalverse ecosystem.

Specifically, the earlier stages (e.g., Stages 1 and 2) may occur in a short interval to establish the foundational capabilities that enable the full realization of the system in Stages 3 and 4, ensuring a cohesive development toward a single inventive concept: a dynamic, multi-generational legacy preservation platform that facilitates therapeutic cross-generational interactions by preserving wisdom across time.

### 5.9.1 Stage 1 (Mournal for Moms, Initial AI Modeling)

This stage introduces the Hybrid Journaling System and VR-enabled Momverse, initiating AI Twin modeling using Eva's Conversational NLP (e.g., BERT/GPT) to capture maternal legacies, such as a mother's story about her child's first steps (**Claim 1(a), Claim 1(c), Claim 5**). This foundational stage sets the groundwork for emotional memory capture and immersive engagement, which are further developed in subsequent stages.

### 5.9.2 Stage 2 (Mournal for Dads, Generational Evolution)

Building on Stage 1, this stage adds Dadverse VR environments and evolves AI Twins via reinforcement learning, enabling paternal mentorship, such as career advice for a descendant (**Claim 1(c), Claim 1(d), Claim 9**). The integration of paternal legacies enhances the system's generational depth, preparing it for broader applicability in the next stages.

### 5.9.3 Stage 3 (Mournal for Individuals, Enhanced Customization)

This stage expands the system to all individuals, incorporating expedited AI Twin creation (Mournal Express) and neuro-interface customization (e.g., Emotiv EPOC) to ensure accessibility for users with diverse needs (**Claims 1, Claim 7, Claim 13**). By building on the capabilities of Stages 1 and 2, this stage broadens the system's reach and adaptability, setting the stage for the fully integrated ecosystem.

### 5.9.4 Stage 4 (Mournalverse, Real-Time Interaction)

At this stage, the system integrates all prior environments and capabilities into the Mournalverse, enabling real-time holographic interactions (e.g., GAN-based facial rendering) and predictive AI-driven mentorship.

The system provides generational users with contextually adaptive conversations with AI Twins, utilizing machine learning models that evolve based on multi-generational input and societal trends (**Claim 1(d), Claim 8, Claim 11, Claim 15**).

This stage transforms the system from a passive legacy storage model into an interactive, evolving digital ancestry system by incorporating:
- **GAN-Based Adaptive Rendering & Sentiment Analysis:** AI Twins dynamically adjust facial expressions, tone, and emotional responses using Generative Adversarial Networks (GANs) to generate realistic facial expressions based on historical data and user inputs, aligning with the recipient's real-time emotional state.
- **Predictive AI Mentorship Engine:** Uses AI-driven life event tracking (e.g., first child, marriage, bereavement) to provide timely, milestone-triggered guidance derived from intergenerational knowledge archives.
- **Multi-User AI Collaboration for Family Legacy Reconstruction:** Multiple heirs can interact simultaneously with AI Twins, contributing to and refining the AI-driven Living Ancestry Tree, ensuring knowledge continuity.
- **Quantum-Secured Legacy Governance:** Legacy content integrity is enforced using tamper-proof encryption and access controls, ensuring AI evolution adheres to user-defined parameters while maintaining privacy.

Mournalverse real-time holographic engagement system ensures:
- **Long-Term AI Evolution:** AI Twins update through multi-generational interaction, preventing knowledge obsolescence.
- **Adaptive Guidance Delivery:** AI mentorship aligns with recipient life stage, emotional state, and societal changes.
- **Ethical & Secure Digital Presence:** AI responses evolve without unauthorized content modification, maintaining authenticity.

This stage establishes a dynamic, AI-driven intergenerational system, enabling real-time, emotionally adaptive legacy preservation, addressing technical challenges related to long-term AI evolution, memory integrity, and predictive AI engagement across generations.

### 5.9.5 Stage 5 (Mournal Lifelong Companion, Child Development and Emotional Support)

The final stage of Mournal's evolution extends the system's capabilities to support a child from birth, with the AI Twin growing alongside them as a lifelong coach, positive influence, and friend (**Claim 1(a), Claim 10, Claim 15).**

The AI Twin leverages emotional intelligence algorithms, sentiment analysis, and generational wisdom to provide AI-driven emotional support and mentorship to foster resilience and personal growth. For example, the AI Twin might encourage a child facing self-doubt before a school presentation by sharing a story of their ancestor's perseverance, adapted to the child's developmental stage, or provide coaching on managing anxiety using therapeutic techniques like guided mindfulness with AI-generated audio prompts synchronized with therapeutic frequencies (e.g., 432 Hz) (**Claim 3, Claim 9).**

This stage ensures that Mournal not only preserves legacies but also actively nurtures the emotional well-being of future generations from their earliest years, creating a lifelong bond between the child and their ancestral wisdom.

Future scalability includes the integration of quantum computing for tamper-proof memory evolution, ensuring data integrity over centuries, unlike traditional encryption methods that are vulnerable to quantum threats (**Claim 1(d)**).

For example, in Stage 4, a descendant receives parenting advice from a holographic AI Twin, with responses adapted via Eva's Predictive Emotional Calibration, while in Stage 5, a child receives real-time emotional coaching from the same AI Twin, demonstrating the non-obvious synergy of the continuous evolution, immersive VR, and quantum security that makes Mournal a system for facilitating therapeutic and secure legacy preservation across generations.

The stagesare a progressive development, but they are not separable inventions; rather, they represent the integrated evolution of the system, with components like quantum-secured storage and holographic interactions working cohesively across the continuum to achieve the unified Mournalverse ecosystem .

### 5.10 User-Configurable Privacy for Locked Content in AI Twin Evolution

The Mournal system includes a novel user-configurable privacy mechanism that allows users to designate certain content, such as videos, audio, or journals resulting from self-reflection, healing, or trapped emotion release, as "locked," meaning it remains inaccessible to pre-authorized descendants **(Claim 1(e)).**

This locked content is stored in the Quantum-Secured Legacy Vault using lattice-based cryptography, ensuring its privacy even against future quantum computing threats (**Claim 1(b), Claim 14**).

Despite being inaccessible to descendants, the locked content is processed by the Adaptive AI Twin using advanced NLP, sentiment analysis, and emotional intelligence algorithms to enhance the AI Twin's emotional authenticity by incorporating private emotional data (**Claim 1(a), Claim 3**).

For example, a user may record a private video reflecting on a traumatic experience, mark it as "visible only to Al Twin," and the AI Twin will use this content to better understand the user's emotional depth and personality, enhancing its interactions with descendants without ever disclosing the locked video.

This feature ensures that users can engage in deep self-reflection and healing while maintaining privacy, yet still pass on their authentic emotional presence to future generations through the AI Twin.

### 5.11 Culturally Sensitive, Ethically Governed AI Across Generations

The Mournal system incorporates an AI-driven cultural sensitivity screening and ethical governance engine that ensures legacy content remains appropriate and respectful across generations (**Claim 1(e), Claim 12, Claim 15**). The cultural sensitivity screening uses NLP and machine learning to analyze content for potential cultural insensitivity, flagging material that may become offensive as societal norms evolve (e.g., language or customs that were acceptable in 2025 but not in 2125).

The ethical governance engine enforces user-defined ethical guidelines (e.g., "do not share content that could cause emotional harm") and adapts these guidelines over time by learning from societal data and descendant interactions, ensuring long-term relevance (**Claim 12**).

The generational adaptation layer enables the AI Twin to evolve its understanding of cultural and ethical norms, ensuring that interactions remain respectful across centuries (**Claim 15**).

For example, if a user's journal entry contains language that later becomes culturally insensitive, the Al will either rephrase the content or withhold it, based on the ethical guidelines, ensuring that descendants experience only respectful and emotionally supportive interactions .

### 5.12 Future Scalability and Technological Integration

The Mournal system is designed with future scalability in mind, enabling its core functionalities, such as holographic AI Twin interactions, immersive 3D legacy environments, and secure data storage, to adapt to emerging technologies, ensuring long-term applicability across generations (**Claim 1, Claim 15**).

This scalability enhances the system's ability to deliver emotionally resonant, therapeutic, and accessible legacy experiences as hardware and software technologies evolve. One aspect of future scalability involves the projection of holographic AI Twins (**Claim 1(d)**).

While current implementations may rely on light-field displays or laser-based holographic systems, advancements in nanotechnology, photonics, and compact energy sources could enable small, portable devices, such as a wristwatch-sized projector; to project high-fidelity holographic AI Twins into the user's environment. This would eliminate the need for bulky equipment, making Mournal's real-time, interactive engagements more seamless and accessible for descendants, allowing them to interact with their ancestors' AI Twins in everyday settings.

Similarly, the immersive 3D experiences provided by the VR Legacy Environments (e.g., Momverse, Dadverse, Mournalverse) can evolve with advancements in augmented reality (AR) hardware (**Claim 1(c)**).

Instead of relying on VR headsets, future implementations could utilize smart glasses with waveguide displays or smart contact lenses equipped with micro-LED displays and eye-tracking sensors to deliver 3D legacy environments directly to the user's field of vision. This would enable descendants to experience the Mournalverse ecosystem as an overlay on their real-world environment, enhancing immersion and integration into daily life.

Beyond hardware advancements, Mournal can integrate with emerging futuristic technologies to further enhance its functionality:
- **Brain-Computer Interfaces (BCIs):** Future BCIs, such as neural implants or advanced non-invasive neural interfaces (e.g., EEG or optogenetics), could allow users to interact with Mournal's AI Twins and 3D environments directly through thought, bypassing traditional input methods like voice or eye-tracking (**Claim 7**). This would enhance accessibility for users with severe physical impairments and create a more intuitive, immersive experience for all users.
- **Ambient AI Integration:** Mournal could integrate with ambient AI systems;networks of sensors, projectors, and AI processors embedded in future environments (e.g., homes, public spaces);to project AI Twins or deliver 3D experiences without user-worn devices, creating a fully immersive "living" legacy environment (**Claim 1(d)**).
- **Synthetic Biology for Data Storage:** Advances in synthetic biology could enable Mournal's legacy data (e.g., memories, AI Twin profiles) to be encoded into DNA or other biological molecules, stored in a bio-integrated system (e.g., a living organism or bio-engineered medium), providing an ultra-long-term, energy-efficient storage solution that complements the Quantum-Secured Legacy Vault (**Claim 1(b)**).
- **AI-Driven Emotional Augmentation via Neuromodulation:** Future neuromodulation technologies (e.g., transcranial magnetic stimulation, neurochemical implants) could allow Mournal's AI to directly influence a user's emotional state during interactions, using AI-driven algorithms to control stimulation parameters for therapeutic safety, enhancing outcomes by triggering neurochemical responses (e.g., dopamine release for comfort) during grief-processing sessions (**Claim 9**).

These future integrations ensure that Mournal remains at the forefront of technological innovation, adapting to advancements in hardware, AI, and biotechnology while continuing to fulfill its mission of preserving wisdom across generations through secure, therapeutic, and accessible interactions.

### 5.13 Example Embodiments

Mournal's functionality is illustrated through the following embodiments, as described in Claims 1-15:
- **Thanksgiving Ancestral Reunion: Real-Time Holographic Interaction Across Generations:** A family gathers for Thanksgiving, and a descendant interacts with holographic projections of ancestors spanning multiple generations, sharing stories, advice, and traditions. The Multi-User Generational AI Twin System reconstructs ancestral personalities in real-time, allowing multiple family members to interact with AI Twins of their great-grandparents, grandparents, and parents in a single immersive session. Emotion-Adaptive AI Twins dynamically adjust speech, tone, and historical insights based on family interactions, ensuring contextually relevant responses. AI Twins share ancestral wisdom, correct misinformation, and even mediate family discussions, reinforcing authentic intergenerational connection, unlike static recorded messages (**Claim 1(d), Claim 9, Claim 15**).
- **Child Meets Their Great-Grandfather: A Dynamic Time-Bridge:** A child, curious about their heritage, asks to meet their great-grandfather who passed away before they were born. The AI Twin Personality Reconstruction Engine recreates the great-grandfather's presence, voice, and speech patterns using AI-driven memory synthesis, historical data analysis, and NLP-based sentiment modeling. Contextual AI Evolution ensures the great-grandfather's AI Twin adapts to the modern world, integrating new historical insights and family knowledge while preserving authentic ancestral wisdom. Emotional AI Synchronization enables the AI Twin to engage in natural dialogue, responding dynamically to the child's curiosity, emotions, and questions, allowing real-time emotional bonding beyond static family records (**Claim 1(a), Claim 1(d), Claim 15**).
- **Wedding Day Message from a Late Parent: Adaptive Sentiment-Based Delivery:** A bride or groom receives a milestone-triggered message from a deceased parent on their wedding day, but only when they are emotionally ready. Eva's Sentiment Readiness Engine ensures the message is not simply delivered on a set date but is unlocked only when the recipient is emotionally prepared. Real-Time Emotional Calibration analyzes the recipient's facial micro-expressions, voice tone, heart rate (via wearables), and text sentiment before delivering the message. If the recipient is overwhelmed, Eva softens delivery, reduces intensity, or postpones the message, ensuring healing instead of triggering grief (**Claim 1(c), Claim 1(e), Claim 4, Claim 11**).
- **Multi-Generational Family Knowledge Exchange: AI Twins Teaching Each Other:** Two AI Twins from different branches of a family tree (e.g., maternal and paternal lineages) interact and share knowledge, ensuring lost traditions are revived. The Cross-Family AI Twin Interaction System enables AI Twins to exchange insights, correct misinformation, and update their stored knowledge bases across generations.

For example, a grandfather's AI Twin shares a long-lost family recipe, while a great-aunt's AI Twin adds historical context about its origins. The recipient can ask both AI Twins questions in real-time, with Eva ensuring privacy-controlled data exchange using blockchain-authenticated access (**Claim 1(d), Claim 15**).
- **Interactive Healing Sessions: AI Twin as a Therapeutic Guide:** A user struggling with unresolved grief engages in a VR-based therapeutic conversation with their late parent's AI Twin, guided by Eva's Conversational Healing Engine.
   Regression-Based Emotional Processing reconstructs specific memories of the deceased parent (e.g., bedtime stories, a childhood hug) based on the user's subconscious emotional needs. Healing Frequencies modulate speech rhythm and voice tonality, synchronizing responses with therapeutic frequencies (e.g., 432 Hz for emotional release).
   The AI Twin acknowledges past misunderstandings, reassures the user, and helps process grief using AI-driven therapeutic conversation models, ensuring emotional relief and healing (**Claim 5, Claim 11, Claim 15**).
- **Future Generations Unlock Ancestral Wisdom: A Time Capsule AI Mentor:** A yet-unborn descendant interacts with an AI Twin recorded by an ancestor centuries earlier, creating a living knowledge lineage. Predictive AI Generational Guidance analyzes historical family patterns and predicts challenges faced by future generations, providing contextually relevant advice even centuries later.
   For example, a 21st-century ancestor records a message for descendants facing global climate change, with the Al Twin updating its wisdom dynamically to ensure relevance.
   Legacy messages are protected using lattice-based cryptography, unlocking only when specific family criteria are met (**Claim 1(b), Claim 3, Claim 14, Claim 15**).
- **Urgent Legacy Preservation Scenario:** A terminally ill user employs Mournal Express to create an AI Twin within 24 hours, recording a message for their child's 18th birthday via voice inputs, delivered via Eva's Predictive Milestone Engine with emotional readiness assessment, addressing delays in conventional systems (**Claim 1(a), Claim 13**).

These embodiments demonstrate the non-obvious synergy of expedited AI Twin creation, immersive holographic interactions, secure cross-family sharing, and therapeutic VR, making Mournal a system for facilitating therapeutic and secure cross-generational legacy preservation.

### 6. Industrial Applicability

The invention, as described in Claims 1-15, is industrially applicable as a software platform for AI-driven dynamic legacy preservation and multi-generational knowledge transfer, integrated with virtual reality (VR) hardware and quantum-secured storage systems. It can be deployed in commercial and institutional settings across multiple technological fields, including artificial intelligence (AI), digital legacy preservation, VR, quantum-secured data storage, and assistive technology. The invention addresses real-world challenges in emotional legacy preservation, secure data governance, and intergenerational mentorship, making it applicable in the following industries:

### 6.1 Digital Legacy & AI-Powered Preservation

The Adaptive AI Twin system (**Claim 1**) ensures that personal wisdom, emotional intelligence, and life experiences are dynamically preserved and continuously evolve posthumously through descendant interactions, using sentiment analysis and reinforcement learning to achieve contextually relevant and emotionally accurate interactions across generations (**Claim 1(a), Claim 10, Claim 13; Section 5.4**). Unlike existing static memorialization tools, which rely on pre-recorded data, the invention enables a self-learning AI system that refines responses over time, making it applicable to digital afterlife services, AI-powered estate planning, and personalized memory archiving.

The Milestone-Based Legacy Vault (**Claim 1(b)**) ensures secure, structured content release based on recipient milestones, further supporting its use in digital legacy platforms. Additionally, the cross-family knowledge sharing system (**Claim 6**) facilitates cultural and historical preservation by enabling AI Twins to integrate and refine familial insights across lineages, making the invention relevant for archival institutions and digital heritage initiatives focused on preserving cultural narratives (**Claim 1(e); Section 5.6).**

### 6.2 Mental Health & Therapeutic Applications

The invention supports grief therapy, emotional healing, and subconscious memory retrieval through the VR-Based Memory Reconstruction and Emotional Healing System (**Claim 1(c)**).

By integrating VR cognitive regression therapy and AI-driven emotional calibration, the system facilitates therapeutic memory reliving, enabling descendants to interact with AI-generated legacy figures for emotional closure (**Claim 9, Claim 15; Section 5.7**).

The Momverse, Dadverse, and Mournalverse VR environments create immersive spaces for legacy exploration and therapeutic engagement, making the invention particularly applicable in bereavement counseling, end-of-life care, and trauma recovery programs. The system's predictive AI-driven generational mentorship, using NLP and machine learning to provide context-aware guidance, further extends its application to psychological resilience training and AI-assisted personal development **(Claim 10, Claim 11; Section 5.4).**

### 6.3 Data Security & Quantum-Resistant Storage

The invention ensures long-term data integrity and security through the Quantum-Secured Legacy Vault (**Claim 1(b)**), which employs lattice-based cryptography, blockchain-integrated storage, and quantum key distribution (QKD) via the BB84 protocol to protect legacy data from unauthorized access and future quantum decryption threats (**Claim 3, Claim 14; Section 5.5**).

These features make the system applicable to:
- Secure digital archiving for legal, estate, and financial records, ensuring long-term data protection.
- Post-quantum data storage solutions for governmental and institutional use, addressing future security challenges.
- Ethical AI governance frameworks ensuring controlled legacy access through biometrically governed, AI-ethical content release, preventing unauthorized posthumous data exploitation **(Claim 1(e); Section 5.5).**

### 6.4 Education & Generational Mentorship

The invention applies to intergenerational knowledge transfer and AI-driven lifelong learning through its Multi-Generational Knowledge Transfer System (**Claim 1(d)**), allowing users to interact with AI-powered representations of ancestors for educational, mentorship, and historical documentation purposes (**Section 5.6**).

This feature is applicable to:
- Family-based mentorship programs where AI Twins provide context-aware, evolving guidance to descendants using transformer-based NLP models (**Claim 11; Section 5.4**).
- Al-assisted educational archives preserving cultural and intellectual contributions for academic and historical research.
- Multi-user collaborative learning in VR-based family storytelling environments, enabling shared educational experiences across generations (**Claim 1(d); Section 5.7**).

### 6.5 Assistive Technology & Accessibility

The invention incorporates inclusive AI-driven interaction through its AI-Powered Accessibility Module (Claim 7), which integrates:
- Multi-modal input systems (voice activation via Google Speech-to-Text API, eye-tracking via Tobii Eye Tracker, EEG neuro-interfaces via Emotiv EPOC) to facilitate interaction for individuals with cognitive and physical impairments.
- Al-driven therapeutic soundscapes (e.g., 432 Hz or 528 Hz binaural beats) for emotional regulation and stress reduction during journaling or VR sessions.
- Caregiver-assisted journaling tools, enabling assisted content management for users with degenerative conditions, such as through voice-activated prompts guided by Eva (**Section 5.8**).

These features extend the invention's applicability to assistive technology, cognitive rehabilitation, and neuroadaptive human-AI interaction frameworks, supporting users in healthcare and accessibility-focused settings (**Claim 13**).

### 6.6 Future Scalability & Emerging Technologies

The invention is scalable for integration with future advancements, including:
- Quantum computing for real-time AI Twin evolution and enhanced predictive modeling, improving the system's ability to adapt to generational changes (**Claim 1(d); Section 5.12**).
- Holographic projection for portable, AI-driven family reunions beyond VR environments, using compact devices like wristwatch-sized projectors (**Claim 1(d); Section 5.12**).
- Brain-computer interfaces (BCI) and synthetic biology for deeper AI interaction models, such as AI-driven emotional augmentation via neuromodulation to enhance therapeutic outcomes **(Section 5.12).**

Beyond its core applications, the system can be utilized in genealogy, child development, and cultural preservation, ensuring ethical, contextually adaptive, and dynamically evolving AI-driven interactions across generations (**Claim 1(e), Claim 10, Claim 15; Section 5.9**).

The invention's integration of secure, emotionally intelligent AI with quantum-secured archival methods and immersive VR experiences positions it to address critical technological and societal needs, making it a versatile solution for industries focused on preserving human heritage, supporting emotional well-being, and fostering continuity across generations.

** References [1] Zhang, L., et al., "Emotion Recognition in Text Using Multimodal Sentiment Analysis," IEEE Transactions on Affective Computing, vol. 14, no. 3, pp. 1234-1245, 2023.

## Claims

1. An AI-driven generational legacy preservation system configured for securely preserving and evolving legacy content across multiple generations by integrating posthumous AI evolution, milestone-based memory unlocking, and quantum-secured legacy governance to enable emotionally intelligent and ethically-governed interactions, comprising:
(a) an Adaptive AI Twin system, including an ethical AI system Eva, configured to dynamically evolve posthumously through descendant interactions, using machine learning to continuously learn and refine its interactions based on multi-generational engagement, while preserving memory integrity through biometric authentication and privacy protocols;
(b) a Milestone-Based AI Memory Release Module integrating biometric authentication, AI-detected emotional readiness assessed via sentiment analysis and behavioral data, and event-triggered content unlocking at predefined life events;
(c) a Quantum-Secured Legacy Vault employing quantum-resistant encryption and AI-regulated posthumous data integrity enforcement to ensure secure storage and access;
(d) an AI-Guided Emotional Memory Structuring System configured to facilitate therapeutic subconscious memory retrieval through conversational AI-driven regression therapy using natural language processing (NLP) algorithms;
(e) an AI-Driven Multi-Generational Knowledge Transfer System enabling real-time AI-Twin mentorship and holographic ancestral interactions for cross-generational knowledge sharing;

2. The system of Claim 1, wherein the Adaptive AI Twin system further comprises:
(a) a biometric identity verification and holographic reconstruction module configured to synthesize user-specific voice, facial expressions, and behavioral characteristics using biometric authentication and machine-learning-driven holographic rendering, ensuring an authentic digital representation;
(b) a memory integrity enforcement system configured to prevent AI-generated responses from altering user-provided memories beyond user-defined parameters, ensuring adherence to original legacy data stored in the Quantum-Secured Legacy Vault;
(c) a posthumous AI evolution governance framework configured with user-defined parameters for posthumous AI evolution, including heir-specific content access restrictions and permanent evolution termination settings;
(d) a Dynamic Emotional Calibration Engine configured to enhance authenticity by adjusting interaction parameters (tone, expression, response content) based on real-time sentiment analysis, recipient life events, and prior engagement history; wherein the system ensures emotionally authentic, ethically-governed interactions while maintaining memory integrity and user-defined control (**Section 5.4**).

3. The system of Claim 1, wherein the Quantum-Secured Legacy Vault further comprises:
(a) a quantum-resistant encryption module configured to secure legacy data using lattice-based cryptography and quantum key distribution, ensuring long-term protection against quantum computing threats with an expected data integrity rate of 99.9%, pending further validation;
(b) a biometric authentication system configured to control access through fingerprint and facial recognition, ensuring authorized access by designated heirs;
(c) a hybrid AI-driven death verification system configured to authenticate a user's passing through multi-source validation, including official records, decentralized notarization, and executor approvals, ensuring tamper-proof posthumous legacy management;
(d) an AI-Enhanced Ethical Unlocking Mechanism configured to ensure milestone-based message access tailored to heir-specific permissions, using machine learning to determine milestone triggers and applying AI-driven ethical controls; wherein the Quantum-Secured Legacy Vault integrates quantum security with AI-driven ethical access to enable secure, ethical, and timely legacy preservation for authorized heirs (**Section 5.5**).

4. The system of Claim 1, wherein the AI-Guided Emotional Memory Structuring System further comprises:
(a) an immersive VR memory reliving system configured to reconstruct memories using 3D rendering, spatial audio, and AI-driven guided prompts, dynamically adjusting based on emotional feedback via sentiment analysis;
(b) an AI-guided emotional processing system integrating NLP-based regression therapy and therapeutic frequencies (e.g., 432 Hz) to facilitate therapeutic subconscious memory retrieval;
(c) a real-time multi-user engagement system enabling synchronized VR interactions among multiple users, enhancing shared memory reliving;
(d) an Interactive Generational Memory Reconstruction Framework configured to enable dynamic family storytelling through VR-based 3D rendering and AI-driven narration; wherein the module integrates emotional processing with immersive memory reliving, fostering therapeutic cross-generational connection (Section **5.7**).

5. The system of Claim 1, wherein the AI-Driven Multi-Generational Knowledge Transfer System further comprises:
(a) a holographic dialogue system configured to simulate AI-generated ancestral personas in real-time holographic projections, using 3D rendering and AI-driven response synthesis;
(b) a dynamic interaction adaptation system using machine learning to refine holographic interactions based on multi-generational engagement;
(c) a Cross-Generational AI Dialogue and Multi-User Collaboration System configured to facilitate adaptive family storytelling and shared holographic or VR familial interactions using NLP, machine learning, and networked 3D rendering to generate context-aware narratives; wherein the system enables immersive, adaptive cross-generational knowledge transfer by leveraging holographic and VR technologies for real-time ancestral interactions (**Section 5.6**).

6. The system of Claim 1, further comprising a Cross-Family AI Twin Knowledge Expansion System for enhancing multi-generational wisdom refinement, comprising:
(a) a cross-family collaboration system configured to facilitate AI-mediated exchanges of family knowledge data between AI Twins of separate lineages using NLP algorithms;
(b) a privacy governance system enforcing privacy during exchanges using differential privacy and quantum-resistant encryption;
(c) an ethical governance system overseeing exchanges with AI-driven bias mitigation and transparency algorithms;
(d) a Living Ancestry Tree system mapping familial relationships using a digital graph database to enable collaborative refinement; wherein the system fosters cross-family connection and collaborative wisdom refinement while ensuring privacy, ethical integrity, and AI-mediated contextual adaptation (**Section 5.6**).

7. The system of Claim 1, further comprising an AI-powered accessibility module for enabling inclusive multi-generational interaction, comprising:
(a) a Multi-Mode Input System supporting voice, eye-tracking, and EEG-based neuro-interfaces (e.g., Emotiv EPOC) for inclusive interaction;
(b) a Healing Frequencies Module providing therapeutic auditory engagement with binaural beats for emotional well-being;
(c) a Caregiver Mode enabling assisted journaling for users with impairments using AI-driven prompts, wherein the module ensures inclusive legacy preservation and emotional support for all users (Section **5.8**).

8. The system of Claim 1, wherein the ethical AI system Eva further comprises:
(a) a Locked Data AI Personality Training system integrating private healing data into AI Twin emotional calibration while protecting it with quantum-resistant encryption;
(b) a Sentiment-Based AI Learning framework ensuring emotionally adaptive interactions using NLP and sentiment analysis;
(c) an AI Twin Interaction Monitoring system preventing emotional distress by analyzing user feedback through real-time emotional monitoring (e.g., facial microexpressions, voice tone) and adjusting interactions; wherein Eva enhances the AI Twin's emotional accuracy and user safety (**Section 5.10**).

9. A method for AI-driven emotional legacy preservation, comprising:
(a) constructing an adaptive AI Twin by processing family knowledge data with NLP, sentiment analysis, and subconscious regression therapy data, secured with quantum-resistant encryption;
(b) performing predictive emotional AI learning to calibrate legacy content delivery based on recipient emotional readiness and milestone triggers, expected to enhance response accuracy by 30%, pending further validation;
(c) calibrating AI responses with ethical governance using quantum-secured biometric authentication and AI-driven bias mitigation;
(d) reconstructing memories in VR to create immersive experiences for emotional legacy preservation; wherein the method ensures secure, ethical, and emotionally resonant multi-generational interactions (**Section 5.7**).

10. A method for interactive generational engagement, comprising:
(a) training Eva to manage AI Twin interactions by integrating historical and descendant feedback using machine learning;
(b) enabling immersive holographic and VR experiences with realistic emotional responsiveness via sentiment analysis and GAN-based rendering;
(c) performing multi-perspective generational storytelling by merging familial recollections into a unified narrative; wherein the method fosters cross-generational connection through adaptive, immersive familial interactions (**Section 5.6**).

11. A method for posthumous wisdom refinement, comprising:
(a) continuously updating AI Twin responses by processing descendant interactions, societal trends, and multi-perspective generational memory using machine learning;
(b) providing heir-specific contextualized AI advisory interactions using NLP and sentiment analysis based on life trajectories and relationship depth;
(c) dynamically updating the Living Ancestry Tree with ongoing generational dialogue and family-driven content enrichment; wherein the method evolves family wisdom and delivers tailored guidance across generations (**Section 5.4**).

12. A method for predictive emotional intelligence and therapeutic memory processing, comprising:
(a) performing adaptive NLP-based emotional tone calibration to ensure compassionate communication;
(b) reconstructing deep-memory emotional experiences using regression-based machine learning and VR-based 3D rendering;
(c) mapping neuro-emotional data through vocal tone, ocular movement, physiological data, and heart rate via wearables to enhance emotional recall accuracy; wherein the method enhances emotional healing through predictive emotional intelligence and therapeutic memory reconstruction (**Section 5.7**).

13. A method for secure ethical AI content management, comprising:
(a) implementing biometric authentication protocols with quantum-resistant encryption for secure access;
(b) performing hybrid death verification using official documentation and executor oversight with quantum-safe authentication;
(c) executing ethical AI content release conditional upon verified milestones and heir identification, with AI-driven ethical governance; wherein the method ensures secure and ethical posthumous sharing of family wisdom (**Section 5.5**).

14. A method for dynamic AI Twin recalibration and accessibility enhancement, comprising:
(a) performing interactive behavioral modeling by analyzing multi-generational descendant interactions;
(b) contextually updating the AI Twin's personality and emotional responses using NLP and sentiment analysis;
(c) providing adaptive accessibility interfaces with voice, eye-tracking, brainwave inputs, therapeutic sound modules (e.g.,432 Hz binaural beats), and caregiver assistance; wherein the method ensures the AI Twin's adaptability and accessibility for all users (**Section 5.8**).

15. A method for quantum-enhanced archival and predictive generational mentorship, comprising:
(a) performing quantum-secured archival by storing emotional data in the Quantum-Secured Legacy Vault using quantum-resistant encryption;
(b) executing predictive AI-driven generational mentoring by analyzing genetic lineage, familial wisdom, historical family patterns, and societal changes;
(c) enabling adaptive, milestone-triggered AI interactions for existing and projected future generations; wherein the method ensures secure preservation and future-oriented sharing of family wisdom (**Section 5.5**).
